(19) Europäisches Patentamt
European Patent Office
Office européen des brevets

(11) Veröffentlichungsnummer: **0 100 038**
**B1**

(12) # EUROPÄISCHE PATENTSCHRIFT

(45) Veröffentlichungstag der Patentschrift: **05.03.86**

(51) Int. Cl.[4]: **C 07 D285/08**, C 07 D285/12, C 07 D417/12// A01N43/82

(21) Anmeldenummer: **83106980.2**

(22) Anmeldetag: **16.07.83**

(54) **Verfahren zur Herstellung von substituierten Thiadiazolyloxyacetamiden.**

(30) Priorität: **28.07.82 DE 3228132**

(43) Veröffentlichungstag der Anmeldung: **08.02.84 Patentblatt 84/06**

(45) Bekanntmachung des Hinweises auf die Patenterteilung: **05.03.86 Patentblatt 86/10**

(84) Benannte Vertragsstaaten: **BE CH DE FR GB IT LI**

(56) Entgegenhaltungen:
**EP-A- 0 018 497**
**EP-A- 0 039 811**
**DE-A- 3 038 635**
**DE-A- 3 038 636**

(73) Patentinhaber: **BAYER AG**
**Konzernverwaltung RP Patentabteilung**
**D-5090 Leverkusen 1 Bayerwerk (DE)**

(72) Erfinder: **Cölln, Reimer, Dr.**
**In den Birken 67**
**D-5600 Wuppertal 1 (DE)**
Erfinder: **Mohrmann, Karl Heinrich, Dr.**
**Roonstrasse 61**
**D-5600 Wuppertal 1 (DE)**

**EP 0 100 038 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein neues Verfahren zur Herstellung von bekannten und neuen, herbizid wirksamen, substituierten Thiadiazolyloxyacetamiden.

Es ist bereits bekannt geworden, daß man herbizid wirksame Azolyloxycarbonsäureamide erhält, wenn man entsprechende α-Hydroxycarbonsäureamide mit entsprechenden Halogenazolen in Gegenwart eines Säureakzeptors umsetzt (vgl. DE-OS 2 914 003, DE-OS 3 038 635 und DE-OS 3 004 326). Zur Anwendung als Säureakzeptoren kamen bisher anorganische Basen, wie z. B. Natriumhydroxid, Kaliumhydroxid, Kaliumcarbonat, Kalium-tertiär-butylat oder Calciumoxid.

Diese Säureakzeptoren eignen sich jedoch zur Herstellung von substituierten Thiadiazolyloxyacetamiden nur unzureichend, da sie durch Begünstigung verschiedener Nebenreaktionen wie z. B. Hydrolyse, N-Alkylierung und Umlagerung zu unbefriedigenden Ausbeuten und uneinheitlichen Endprodukten führen.

Es wurde nun gefunden, daß man substituierte Thiadiazolyloxyacetamide der allgemeinen Formel (I),

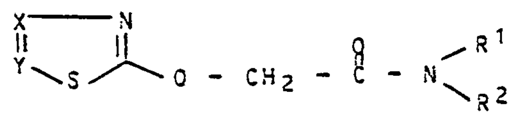
(I)

in welcher

$R^1$ und $R^2$ unabhängig voneinander für Wasserstoff, Alkyl, Alkenyl, Alkinyl, für gegebenenfalls substituiertes Cycloalkyl oder Cycloalkenyl, für Halogenalkyl, Alkoxyalkyl und Alkoxy, für Aralkyl und gegebenenfalls substituiertes Aryl stehen, oder

$R^1$ und $R^2$ zusammen mit dem Stickstoffatom, an welches sie gebunden sind, für einen gegebenenfalls substituierten, gesättigten oder ungesättigten Heterocyclus stehen, der weitere Heteroatome enthalten kann, und

X und Y jeweils alternativ für ein Stickstoffatom und die C-$R^3$-Gruppierung stehen, wobei

$R^3$ für Alkyl, Alkoxy, Alkylthio, Alkylsulfonyl, Halogenalkyl, Aralkyl, Aralkoxy, Aralkylthio oder gegebenenfalls substituiertes Aryl steht,

durch Umsetzung von substituierten 5-Halogenthiadiazolen der Formel (II)

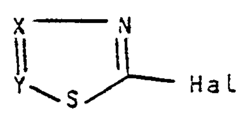
(II)

in welcher

X und Y die oben angegebene Bedeutung haben und

Hal für Halogen steht,

mit Hydroxyacetamiden der Formel (III)

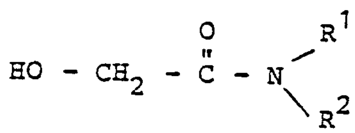
(III)

in welcher $R^1$ und $R^2$ die oben angegebene Bedeutung haben, in Gegenwart einer Base als Säureakzeptor und gegebenenfalls in Gegenwart eines Verdünnungsmittels dann in besonders guten Ausbeuten und hoher Reinheit erhält, wenn man als Base Lithiumhydroxid oder dessen Hydrat einsetzt und bei Temperaturen zwischen − 10 °C und + 60 °C arbeitet.

Es ist als ausgesprochen überraschend zu bezeichnen, daß die Verwendung von Lithiumhydroxid als Säurebindemittel bei der erfindungsgemäßen Umsetzung — im Vergleich zur vorbekannten Verfahrensweise — zu beträchtlich gesteigerten Ausbeuten und zu Produkten von erheblich verbesserter Reinheit führt, weil man im Hinblick auf den Stand der Technik erwarten mußte, daß die Verwendung von Alkalihydroxiden als Säurebindemittel im Fall von substituierten Thiadiazolyloxyacetamiden nur unbefriedigende Ausbeuten und unreine Produkte ergibt (vgl. DE-OS 3 004 326).

Nach dem erfindungsgemäßen Verfahren erhält man vorzugsweise Verbindungen der Formel (I), in welcher

$R^1$ und $R^2$ unabhängig voneinander für Wasserstoff, geradkettiges oder verzweigtes Alkyl mit 1 bis 8 Kohlenstoffatomen, geradkettiges oder verzweigtes Alkenyl und Alkinyl mit jeweils 2 bis 8 Kohlenstoffatomen, für gegebenenfalls einfach oder mehrfach, gleich oder verschieden substituiertes Cycloalkyl oder Cycloalkenyl mit jeweils 3 bis 7 Kohlenstoffatomen, wobei als Substituenten insbesondere Alkylreste mit 1 bis 4 Kohlenstoffatomen infrage kommen, für geradkettiges oder verzweigtes Alkoxy und Alkoxyalkyl mit 1 bis 8 Kohlenstoffatomen, für Halogenalkyl mit 1 bis 8 Kohlenstoffatomen und 1 bis 5 Halogenatomen, insbesondere Fluor, Chlor und Brom, für Aralkyl mit 6 bis 10 Kohlenstoffatomen im

Arylteil und 1 bis 2 Kohlenstoffatomen im Alkylteil, sowie für gegebenenfalls einfach oder mehrfach, gleich oder verschieden substituiertes Aryl mit 6 bis 10 Kohlenstoffatomen steht, wobei als Substituenten infrage kommen : Halogen, geradkettiges oder verzweigtes Alkyl, Alkoxy oder Alkylthio mit jeweils 1 bis 4 Kohlenstoffatomen, Halogenalkyl, Halogenalkoxy und Halogenalkylthio mit jeweils 1 bis 2 Kohlenstoffatomen und 1 bis 5 Halogenatomen, insbesondere Fluor, Chlor oder Brom, sowie Nitro, oder

$R^1$ und $R^2$ zusammen mit dem Stickstoffatom, an das sie gebunden sind, für einen gegebenenfalls einfach oder mehrfach, gleich oder verschieden substituierten gesättigten oder ungesättigten, 5- bis 7-gliedrigen Heterocyclus stehen, der bis zu 2 weitere Heteroatome, insbesondere Stickstoff und Sauerstoff enthalten kann, wobei als Substituenten infrage kommen : geradkettiges oder verzweigtes Alkyl mit 1 bis 6 Kohlenstoffatomen, auch in Form eines anellierten Ringsystemes, Aryl mit 6 bis 10 Kohlenstoffatomen, auch in Form eines anellierten Ringsystemes oder Dioxyalkylen mit 2 bis 3 Kohlenstoffatomen, sowie

X und Y die in der Erfindungsdefinition angegebene Bedeutung haben, wobei

$R^3$ vorzugsweise für geradkettiges oder verzweigtes Alkyl, Alkoxy, Alkylthio und Alkylsulfonyl mit jeweils 1 bis 6 Kohlenstoffatomen, für Halogenalkyl mit 1 bis 6 Kohlenstoffatomen und 1 bis 5 Halogenatomen, insbesondere Fluor, Chlor und Brom, für Aralkyl, Aralkoxy und Aralkylthio mit 6 bis 10 Kohlenstoffatomen im Arylteil und 1 bis 2 Kohlenstoffatomen im Alkylteil sowie für gegebenenfalls einfach oder mehrfach gleich oder verschieden substituiertes Aryl mit 6 bis 10 Kohlenstoffatomen steht, wobei als Substituenten infrage kommen : Alkyl und Alkoxy mit jeweils 1 bis 4 Kohlenstoffatomen, Halogenalkyl mit 1 bis 4 Kohlenstoffatomen und 1 bis 5 Halogenatomen insbesondere Fluor, Chlor oder Brom.

Das erfindungsgemäße Verfahren betrifft besonders bevorzugt Verbindungen der Formel (I), in welcher

$R^1$ und $R^2$ unabhängig voneinander für Wasserstoff, für geradkettiges oder verzweigtes Alkyl mit 1 bis 6 Kohlenstoffatomen, für geradkettiges oder verzweigtes Alkenyl und Alkinyl mit jeweils 2 bis 6 Kohlenstoffatomen, gegebenenfalls ein- bis dreifach, gleich oder verschieden durch Methyl oder Ethyl substituiertes Cycloalkyl bzw. Cycloalkenyl mit 5 bis 7 Kohlenstoffatomen, für verzweigtes oder geradkettiges Alkoxy und Alkoxyalkyl mit 1 bis 6 Kohlenstoffatomen, für Halogenalkyl mit 1 bis 6 Kohlenstoffatomen und 1 bis 5 Halogenatomen, insbesondere Fluor, Brom und Chlor, für Benzyl sowie für gegebenenfalls ein- bis dreifach gleich oder verschieden substituiertes Phenyl, wobei als Substituenten besonders bevorzugt sind : Methyl, Ethyl, Methoxy, Methylthio, Trifluormethyl, Trifluormethoxy, Trifluormethylthio, Fluor, Chlor, Nitro, oder

$R^1$ und $R^2$ zusammen mit dem Stickstoffatom, an das sie gebunden sind, für die gegebenenfalls ein- bis dreifach, gleich oder verschieden substituierten Heterocyclen

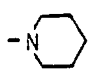 ; 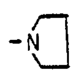 ; 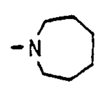 ; 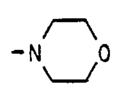 ; 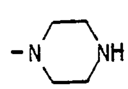 ;

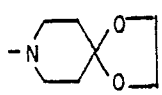 ; 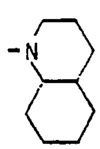 ; 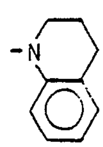

stehen, wobei als Substituenten besonders bevorzugt sind : Methyl, Ethyl und Phenyl, sowie

X und Y die in der Erfindungsdefinition angegebene Bedeutung haben, wobei

$R^3$ besonders bevorzugt für geradkettiges oder verzweigtes Alkyl, Alkoxy, Alkylthio oder Alkylsulfonyl mit jeweils 1 bis 4 Kohlenstoffatomen, für Halogenalkyl mit 1 bis 2 Kohlenstoffatomen und 1 bis 5 gleichen oder verschiedenen Halogenatomen, insbesondere Fluor, Chlor oder Brom, für Benzyl oder Benzylthio sowie für gegebenenfalls ein- bis dreifach gleich oder verschieden substituiertes Phenyl mit den besonders bevorzugten Substituenten : Methyl, Methoxy und Trifluormethyl, steht.

Verwendet man als Ausgangsstoffe beispielsweise 3-Dichlorfluormethyl-5-chlor-1,2,4-thiadiazol und Hydroxyessigsäure-N-methylanilid sowie 4-Methylpentan-2-on als Lösungsmittel und Lithiumhydroxidhydrat als Säurebindemittel, so kann die beim erfindungsgemäßen Verfahren ablaufende Reaktion durch folgendes Formelschema dargestellt werden :

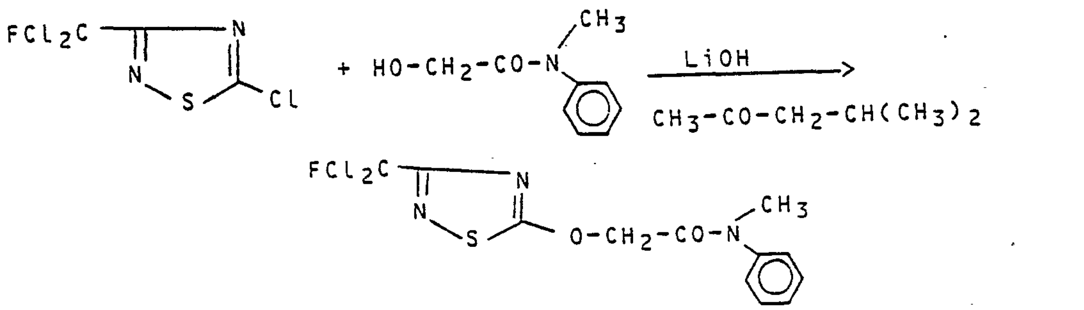

Die beim erfindungsgemäßen Verfahren als Ausgangsstoffe zu verwendenden 5-Halogenthiadiazole sind durch die Formel (II) allgemein definiert. In dieser Formel stehen X und Y vorzugsweise für diejenigen Reste, welche bei der Beschreibung der entsprechenden Reste in Formel (I) als bevorzugt angegeben wurden. Hal steht bevorzugt für Fluor, Chlor oder Brom.

Als Beispiele für die Ausgangsverbindungen der Formel (II) seien genannt :

3-Methyl-, 3-Ethyl-, 3-n-Propyl-, 3-Isopropyl-, 3-t-Butyl, 3-Dichlorfluormethyl-, 3-Difluorchlormethyl-, 3-Trifluormethyl-, 3-Trichlormethyl-, 3-Methylthio-, 3-Benzylthio-, 3-Methylsulfonyl-5-chlor-1,2,4-thiadiazol, -5-brom-1,2,4-thiadiazol und -5-fluor-1,2,4-thiadiazol,

2-Methyl-, 2-Ethyl-, 2-n-Propyl-, 2-Isopropyl-, 2-t-Butyl-, 2-Methylthio-, 2-Ethylthio-, 2-n-Propylthio-, 2-n-Butylthio-, 2-Methylsulfonyl-, 2-Ethylsulfonyl-, 2-n-Propylsulfonyl-, 2-Phenyl-, 2-Trifluormethyl-, 2-Trichlormethyl-, 2-Difluorchlormethyl-, 2-Dichlorfluormethyl-, -5-chlor-1,3,4-thiadiazol, -5-brom-1,3,4-thiadiazol und -5-fluor-1,3,4,-thiadiazol,

3-(3-Trifluormethyl-phenyl)- und 3-(4-Trifluormethylphenyl)-5-fluor-, -5-chlor- und -5-brom-1,2,4-thiadiazol, 2-Phenyl-, 2-(2-Trifluormethylphenyl)-, 2-(3-Trifluormethylphenyl)-, 2-(4-Trifluormethylphenyl)-, 2-(2-Methoxymethylphenyl)-, 2-(3-Methoxymethylphenyl)-5-fluor-, -5-chlor- und -5-brom-1,3,4-thiadiazol.

Die Verbindungen der Formel (II) sind bekannt oder können nach an sich bekannten Verfahren auf einfache Weise hergestellt werden (vgl. z. B. DE-OS 3 004 326 ; J. org. Chem. *27*, 2589-2592 (1962) ; US-Patent 3 260 588).

Die weiterhin als Ausgangsstoffe beim erfindungsgemäßen Verfahren zu verwendenden Hydroxyacetamide sind durch die Formel (III) allgemein definiert. In dieser Formel stehen $R^1$ und $R^2$ vorzugsweise für diejenigen Reste, welche bei der Beschreibung der entsprechenden Reste in Formel (I) als bevorzugt angegeben wurden.

Als Beispiele für die Ausgangsverbindungen der Formel (III) seien genannt :

Hydroxyessigsäure-methylamid, -ethylamid, n-propylamid, -iso-propylamid, -n-butylamid, -iso-butylamid, -dimethylamid, -diethylamid, -di-n-propylamid, -di-iso-propylamid, -di-n-butylamid, -diisobutylamid, -N-methyl-N-n-propyl-amid, -N-methyl-N-n-butylamid, -N-methyl-N-iso-propyl-amid, -N-methyl-N-isobutylamid, -N-methyl-N-t-butyl-amid, -N-methyl-N-sek.-butyl-amid, -N-ethyl-N-n-propyl-amid, -N-ethyl-N-isopropylamid, -N-ethyl-N-n-butylamid, -N-ethyl-N-isobutylamid, -N-ethyl-sek.-butylamid, -N-ethyl-tert.-butylamid, -N-n-propyl-iso-propylamid, -N-n-propyl-n-butylamid, -N-n-propyl-iso-butylamid, -N-n-propyl-sek.-butylamid, N-n-propyl-tert.-butylamid, -N-n-butyl-iso-butylamid, -N-n-butyl-sek.-butylamid, -N-n-butyl-tert.-butylamid, -di-(2-methoxy-ethyl)-amid, di-allyl-amid, -N-methyl-N-propargyl-amid, -N-methyl-N-(1-methyl-propargyl)-amid, -dipropargyl-amid, -cyclopentyl-amid, -N-methyl-N-cyclopentyl-amid, -cyclohexyl-amid, -N-methyl-N-cyclohexyl-amid, -N-methyl-(1,1-dimethyl-propargyl)-amid, -N-methyl-(2,2,2-trifluorethyl)-amid, -N-ethyl-cyclohexylamid, -anilid, -2-nitro-, -3-nitro- und 4-nitro-phenyl-amid, -2-chlor-, 3-chlor- und -4-chlor-phenyl-amid, -2,4-dichlor-, 2,5-dichlor-, 3,4-dichlor- und 3,5-dichlor-phenyl-amid, -2-methyl-, -3-methyl- und -4-methyl-phenyl-amid, -N-methylanilid, -N-methyl-N-(2-nitro-phenyl)-, N-methyl-N-(3-nitro-phenyl)-, -N-methyl-N-(4-nitro-phenyl)-amid, -N-methyl-N-(2-chlor-phenyl)-, -N-methyl-N-(3-chlor-phenyl)-, -N-methyl-N-(4-chlor-phenyl)-amid, -N-methyl-N-(3-nitro-6-methyl-phenyl)-amid, -N-methyl-N-(4-chlor-3-methyl-phenyl)-amid, -N-methyl-N-(3,5-trifluormethyl-phenyl)-amid, -N-methyl-N-(4-methoxy-phenyl)-amid, -N-ethyl-anilid, -N-ethyl-N-(2-nitro-phenyl)-, -N-ethyl-N-(3-nitro-phenyl)-, -N-ethyl-N-(4-nitro-phenyl)-amid, -N-ethyl-N-(2-chlor-phenyl)-, -N-ethyl-N-(3-chlor-phenyl)-, -N-ethyl-N-(4-chlor-phenyl)-amid, -N-ethyl-N-(3-nitro-6-methyl-phenyl)-amid, -N-propyl-anilid, -N-propyl-N-(2-nitro-phenyl)-, -N-propyl-N-(3-nitro-phenyl)-, N-propyl-N-(4-nitro-phenyl)-amid, -N-Propyl-N-(2-chlor-phenyl)-, -N-propyl-N-(3-chlor-phenyl)-, -N-propyl-N-(4-chlor-phenyl)-amid, -N-propyl-N-(2-methyl-phenyl)-, -N-propyl-N-(3-methyl-phenyl)-, -N-propyl-N-(4-methyl-phenyl)-amid, -N-propyl-N-(3-nitro-6-methyl-phenyl)-amid, -N-isopropylanilid, -N-isopropyl-N-(2-methyl-phenyl)-, -N-iso-propyl-N-(3-methyl-phenyl)-, -N-iso-propyl-N-(4-methyl-phenyl)-amid, -N-iso-propyl-N-(3-nitro-6-methyl-phenyl)-amid, -N-butyl-anilid, -N-butyl-N-(2-nitro-phenyl)-, -N-butyl-N-(3-nitro-phenyl)-, -N-butyl-N-(4-nitro-phenyl)-amid, -N-butyl-N-(2-chlorphenyl)-, -N-butyl-N-(3-chlor-phenyl)-, -N-butyl-N-(4-chlor-phenyl)-amid, -N-butyl-N-(2-methyl-phenyl)-, -N-butyl-N-(3-methyl-phenyl)-, -N-butyl-N-(4-methyl-phenyl)-amid, -N-butyl-N-(3-nitro-6-methyl-phenyl)-amid, -N-isobutyl-anilid, -N-iso-butyl-N-(2-nitro-phenyl)-, -N-iso-butyl-N-(3-nitro-phenyl)-, -N-iso-butyl-N-(4-nitro-phenyl)-amid, -N-iso-butyl-N-(2-chlor-phenyl)-, -N-iso-butyl-N-(3-chlor-phenyl)-, -N-iso-butyl-N-(4-chlor-phenyl)-amid, -N-isobutyl-N-(2-methyl-phenyl)-, -N-iso-butyl-N-(3-methyl-phenyl)-, N-iso-butyl-N-(4-methyl-phenyl)-amid, -N-iso-butyl-N-(3-nitro-6-methyl-phenyl)-amid, -benzylamid, -dibenzyl-amid, -N-methyl-N-benzylamid, -N-ethyl-N-benzylamid, -N-propyl-N-benzylamid, -N-butyl-N-benzylamid, -pyrrolidid, -2-methyl-pyrrolidid, -morpholid, -piperidid, -2-methyl-piperidid, -4-methyl-piperidid, -2,4-dimethyl-piperidid, -2,4,6-trimethyl-piperidid, -2-ethyl-piperidid, -4-ethyl-piperidid, -2,4-diethyl-piperidid, -2,4,6-triethyl-piperidid, -2-methyl-4-ethyl-piperidid, -2-ethyl-4-methyl-piperidid, -2-methyl-5-ethyl-piperidid, -2-ethyl-5-methyl-piperidid, -2-methyl-6-ethyl-piperidid, -1,2,3,4-tetrahydrochinolid, -2-methyl-1,2,3,4-tetrahydrochinolid, -perhydrochinolid, -2-methyl-perhydrochinolid, -3-methyl-piperidid, -3,5-dimethyl-piperidid, -3,5-diethylpiperidid, -N-methyl-N-(2-methylthiophenyl)-, -N-methyl-N-(3-methylthiophenyl)- und N-Methyl-N-(4-methylthiophenyl)-amid, -N-methyl-N-(2-fluorphenyl)-, -N-methyl-N-(3-fluorphenyl)- und -N-methyl-N-(4-fluorphenyl)-amid, -N-methyl-N-(2-trifluor-methylphenyl)-, -N-methyl-N-(3-trifluormethylphenyl)- und -N-methyl-N-(4-trifluormethylphenyl)-amid, -N-methyl-N-(2-trifluormethoxyphenyl)-, -N-methyl-N-methyl-N-(3-trifluor-methoxyphenyl)- und -N-methyl-N-(4-trifluormethoxyphenyl)-amid, -N-methyl-N-(2-trifluormethylthiophenyl)-, -N-methyl-N-(3-trifluormethylthiphenyl)- und -N-methyl-N-(4-trifluormethylthiophenyl)-amid, -N-methyl-N-methylenmethoxyamid, -N-methyl-N-cyclohex-1-enylamid, -N-methyl-N-(3,5,5-trimethylcyclohex-1-enyl)-amid, -6-methylperhydrochinolid, -N-methyl-N-(2-methylphenyl)-, -N-methyl-N-(3-methylphenyl)- und N-methyl-N-(4-methylphenyl)-amid, -N-methyl-N-(2,3-dimethylphenyl)- und -N-methyl-N-(2,4-dimethylphenyl)-amid, -N-methyl-N-(2-methoxyphenyl)- und -N-methyl-N-(3-methoxy-phenyl)-amid, -N-methyl-N-(2,4-dichlorphenyl)- und -N-methyl-N-(3,4-dichlorphenyl)-amid, -N-ethyl-N-(2-methylphenyl)-, -N-ethyl-N-(3-methyl-phenyl)- und -N-ethyl-N-(4-methylphenyl)-amid, -N-benzyl-anilid, -N-benzyl-N-propylamid, -N-ethyl-N-(2,2,2-trifluorethyl)-amid, N,N-di-(2-methoxyethyl)-amid, -N-methoxy -N-butylamid, -N-methyl-N-(2-methylperhydrofuran-2-yl)-methyl-amid, -perhydroazepid, -3-ethyl-piperidid, -4-phenyl-1,4-piperazid, -3,3,5-trimethyl-perhydroazepid, -4,4-dioxyethylen-piperidid, -3-methyl-morpholid und -3,5-dimethylmorpholid.

Die Verbindungen der Formel (III) sind bekannt oder können nach an sich bekannten Verfahren auf einfache Weise hergestellt werden. (vgl. z. B. DE-OS 3 004 326 ; EP-PA 5501 ; DE-OS 2 904 490 ; US-PS 3 399 988 ; DE-OS 2 201 432 ; DE-OS 2 647 481).

Das erfindungsgemäße Verfahren wird vorzugsweise in Gegenwart eines geeigneten Verdünnungsmittels durchgeführt. Als solche kommen praktisch alle inerten organischen Solventien infrage. Hierzu gehören insbesondere Alkohole, wie Methanol, Ethanol, n- und iso-Propanol, n-, iso-, sek.- und tert.-Butanol, Ether wie Diethylether, Dibutylether, Tetrahydrofuran und Dioxan, Ketone wie Aceton, Methylethylketon, Methylisopropylketon und Methylisobutylketon, Nitrile wie Acetonitril und Propionsäurenitril, sowie die hochpolaren Lösungsmittel Dimethylformamid, Dimethylsulfoxid, Sulfolan und Hexamethylphosphorsäuretriamid.

Die Reaktionstemperatur wird bei dem erfindungsgemäßen Verfahren im allgemeinen zwischen − 10 °C und + 60 °C, vorzugsweise zwischen + 20 °C und + 40 °C, gehalten. Das Verfahren wird im allgemeinen bei Normaldruck durchgeführt.

Zur Durchführung des erfindungsgemäßen Verfahrens setzt man pro Mol 5-Halogenthiadiazol der Formel (II) 0,9 bis 1,2 Mol Hydroxyacetamid der Formel (III) und 0,9 bis 1,2 Mol Lithiumhydroxid bzw. dessen Hydrat ein. Vorzugsweise verwendet man äquimolare Mengen von allen drei Komponenten.

In einer bevorzugten Ausführungsform des erfindungsgemäßen Verfahrens legt man die Ausgangsverbindungen der Formeln (II) und (III) in einem Lösungsmittel gelöst vor und gibt das Lithiumhydroxid bzw. dessen Hydrat portionsweise zu. Eine alternative Ausführungsform besteht darin, daß man das Hydroxyessigsäureamid der Formel (III) zusammen mit dem Lithiumhydroxid bzw. dessen Hydrat in dem entsprechenden Lösungsmittel vorlegt und die Thiadiazolkomponente der Formel (II) portionsweise zugibt.

Das Reaktionsgemisch wird darauf in beiden Ausführungsformen bis zum Ende der Umsetzung bei der bevorzugten Reaktionstemperatur gerührt und dann wie üblich aufgearbeitet.

Die nach dem erfindungsgemäßen Verfahren herzustellenden bekannten Verbindungen der Formel (I) besitzen bekanntermaßen herbizide Eigenschaften (vgl. z. B. DE-OS 3 004 326) ; die hiernach erhältlichen neuen Verbindungen wirken in grundsätzlich gleicher Weise.

## Beispiel 1

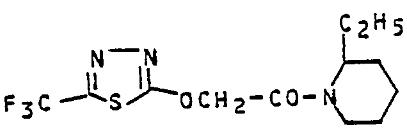

Man legt 171,2 g (1 Mol) Hydroxyessigsäure-2-ethyl-piperidid und 188,6 g (1 Mol) 2-Trifluormethyl-5-chlor-1,3,4-thiadiazol, gelöst in 800 ml Methyl-isobutylketon, vor und gibt unter Rühren im Laufe von 30 bis 45 Minuten bei 20-25 °C 43,5 g (1 Mol) Lithiumhydroxidhydrat portionsweise hinzu. Der Ansatz wird eine Stunde lang bei 20-25 °C nachgerührt.

Man setzt dann noch 200 ml des Lösungsmittels und 1 l Wasser hinzu und trennt die Phasen. Die organische Phase wird neutral gewaschen und unter vermindertem Druck bei einer Badtemperatur von maximal 40 °C vom Lösungsmittel befreit. Man erhält 288,9 g (89,4 % der Theorie) an 2-Trifluormethyl-1,3,4-thiadiazol-5-yl-oxyessigsäure-2-ethyl-piperidid in Form eines klaren hellgelben Öls vom Brechungsindex $n_D^{20} = 1{,}4867$. Das Reaktionsprodukt weist nach Analyse (HPLC) einen Gehalt von 85 % auf.

## Beispiel 2

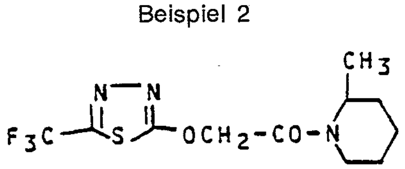

Man legt 157,2 g (1 Mol) Hydroxyessigsäure-2-methyl-piperidid und 188,6 g (1 Mol) 2-Trifluormethyl-5-chlor-1,3,4-thiadiazol, gelöst in 800 ml Methyl-isobutylketon, vor und gibt unter Rühren im Laufe von ca. 45 Minuten bei 20-25 °C 43,5 g (1 Mol) Lithiumhydroxidhydrat portionsweise hinzu.

Der Ansatz wird 1 Stunde lang bei 20-25 °C nachgerührt. Man setzt dann noch 200 ml des Lösungsmittels und 1 l Wasser hinzu, verrührt und trennt die Phasen.

Die organische Phase wird neutral gewaschen und unter vermindertem Druck bei einer Badtemperatur von maximal 40 °C vom Lösungsmittel befreit. Man erhält 275,4 g (89 % der Theorie) 2-Trifluormethyl-1,3,4-thiadiazol-5-yl-oxyessigsäure-2-methylpiperidid in Form eines orangefarbenden Öls vom Brechungsindex $n_D^{20}$ = 1,480 3.

Beispiel 3

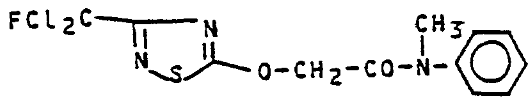

Zu 16,5 g (0,1 Mol) Hydroxyessigsäure-N-methyl-anilid und 16,6 g (0,075 Mol) 3-Dichlorfluormethyl-5-chlor-1,2,4-thiadiazol in 80 ml Methyl-isobutyl-keton gibt man im Laufe von 30 Minuten bei 25-30 °C portionsweise 4,4 g Lithiumhydroxidhydrat (0,1 Mol) und läßt die Mischung 2 1/2 Stunden bei 20-25 °C nachrühren.

Man wäscht dann die organische Phase neutral und entfernt das Lösungsmittel unter vermindertem Druck bei einer maximalen Badtemperatur von 50 °C. Der beim Abkühlen kristallisierende Rückstand wird aus 30 ml Ligroin umkristallisiert. Man erhält 24,3 g (92,5 % der Theorie) an 3-Dichlorfluormethyl-1,2,4-thiadiazol-5-yl-oxyessigsäure-N-methylanilid in Form farbloser Kristalle vom Schmelzpunkt 71-73 °C.

Beispiel 4

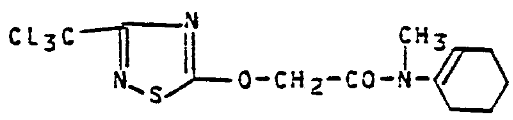

Zu 16,7 g (0,07 Mol) 3-Trichlormethyl-5-chlor-1,2,4-thiadiazol und 11,8 g (0,07 Mol) Hydroxyessigsäure-N-methyl-N-cyclohex-1-enylamid in 100 ml Methyl-isobutyl-keton gibt man portionsweise bei 0 °C 3,1 g (0,07 Mol) Lithiumhydroxidhydrat und läßt die Mischung 30 Minuten bei 0 °C und anschließend über Nacht bei 25 °C rühren. Nach Zugabe von Wasser wird das fest ausfallende Reaktionsprodukt abgesaugt, gewaschen und getrocknet. Man erhält 22,0 g (84,8 % der Theorie) an 3-Trichlormethyl-1,2,4-thiadiazol-5-yl-oxyessigsäure-N-methyl-N-cyclohexen-1-yl-amid in Form beigefarbener Kristalle vom Schmelzpunkt 78 °C.

Vergleichsbeispiel A

(Verwendung von NaOH)

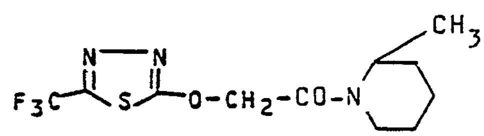

Man legt 15,7 g (0,1 Mol) Hydroxyessigsäure-2-methyl-piperidid und 18,9 g (0,1 Mol) 2-Trifluormethyl-5-chlor-1,3,4-thiadiazol, gelöst in 80 ml Methyl-isobutyl-keton, vor und gibt unter Rühren in Laufe von 20 Minuten bei 20-25 °C 4,5 g Natriumhydroxid in Schuppenform portionsweise hinzu und läßt die Mischung 1 Stunde lang bei 20-25 °C nachrühren.

Man setzt noch 20 ml des Lösungsmittels und 100 ml Wasser hinzu und trennt die Phasen. Die organische Phase wird neutral gewaschen und unter vermindertem Druck bei einer Badtemperatur von maximal 40 °C vom Lösungsmittel befreit. Man erhält 25,0 g eines dunklen viskosen Öls, das nach Analyse (HPLC) jedoch nur einen Gehalt von 62,7 % an 2-Trifluormethyl-1,3,4-thiadiazol-5-yl-oxyessigsäure-2-methylpiperidid aufweist.

Vergleichsbeispiel B

(Verwendung von NaOH)

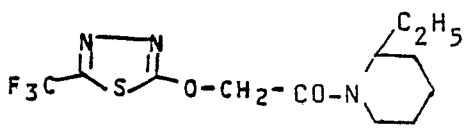

Man legt 17,1 g (0,1 Mol) Hydroxyessigsäure-2-ethyl-piperidid und 18,9 g (0,1 Mol) 2-Trifluormethyl-5-chlor-1,3,4-thiadiazol, gelöst in 80 ml Methyl-isobutyl-keton vor und gibt unter Rühren im Laufe von 20 Minuten bei 20 bis 25 °C, 4,5 g Natriumhydroxid in Schuppenform portionsweise hinzu und läßt die Mischung 1 Stunde lang bei 20 bis 25 °C nachrühren.

Man setzt noch 20 ml des Lösungsmittels und 100 ml Wasser hinzu und trennt die Phasen. Die organische Phase wird neutral gewaschen und unter vermindertem Druck bei einer Badtemperatur von maximal 40 °C vom Lösungsmittel befreit. Man erhält 26,7 g eines dunklen Öles, das nach Analyse (HPLC) jedoch nur einen Gehalt von 62,6 % an 2-Trifluormethyl-1,3,4-thiadiazol-5-yl-oxyessigsäure-2-ethyl-piperidid aufweist.

## Patentansprüche

1. Verfahren zur Herstellung von substituierten Thiadiazolyloxyacetamiden der allgemeinen Formel (I),

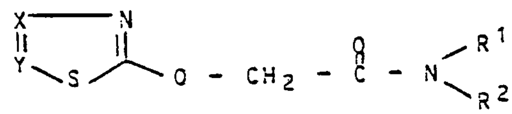

(I)

in welcher

$R^1$ und $R^2$ unabhängig voneinander für Wasserstoff, Alkyl, Alkenyl, Alkinyl, für gegebenenfalls substituiertes Cycloalkyl oder Cycloalkenyl, für Halogenalkyl, Alkoxyalkyl und Alkoxy, für Aralkyl und gegebenenfalls substituiertes Aryl stehen, oder

$R^1$ und $R^2$ zusammen mit dem Stickstoffatom an welches sie gebunden sind, für einen gegebenenfalls substituierten, gesättigten oder ungesättigten Heterocyclus stehen, der weitere Heteroatome enthalten kann, und

X und Y jeweils alternativ für ein Stickstoffatom und die C-$R^3$-Gruppierung stehen, wobei

$R^3$ für Alkyl, Alkoxy, Alkylthio, Alkylsulfonyl, Halogenalkyl, Aralkyl, Aralkoxy, Aralkylthio oder gegebenenfalls substituiertes Aryl steht,

durch Umsetzung von substituierten 5-Halogenthiadiazolen der Formel (II)

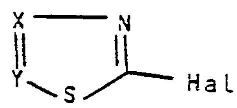

(II)

in welcher

X und Y die oben angegebene Bedeutung haben und

Hal für Halogen steht,

mit Hydroxyacetamiden der Formel (III)

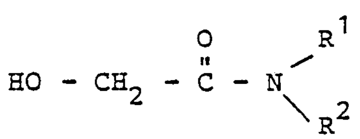

(III)

in welcher $R^1$ und $R^2$ die oben angegebene Bedeutung haben, in Gegenwart einer Base als Säureakzeptor und gegebenenfalls in Gegenwart eines Verdünnungsmittels, dadurch gekennzeichnet, daß man als Base Lithium-hydroxid oder dessen Hydrat einsetzt und bei Temperaturen zwischen − 10 °C und + 60 °C arbeitet.

2. Verfahren nach Anspruch 1, dadurch gekennzeichnet, daß man die Umsetzung bei Temperaturen zwischen + 20 °C und + 40 °C durchführt.

3. Verfahren nach Anspruch 1, dadurch gekennzeichnet, daß man pro Mol 5-Halogenthiadiazol der Formel (II) 0,9 bis 1,2 Mol Hydroxyacetamid der Formel (III) und 0,9 bis 1,2 Mol Lithiumhydroxid bzw. dessen Hydrat einsetzt.

4. Verfahren nach Anspruch 1, dadurch gekennzeichnet, daß man 2-Trifluormethyl-5-chlor-1,3,4-thiadiazol mit Hydroxyessigsäure-2-ethyl-piperidid umsetzt.

5. Verfahren nach Anspruch 1, dadurch gekennzeichnet, daß man 2-Trifluormethyl-5-chlor-1,3,4-thiadiazol mit Hydroxyessigsäure-2-methyl-piperidid umsetzt.

6. Verfahren nach Anspruch 1, dadurch gekennzeichnet, daß man 3-Dichlorfluormethyl-5-chlor-1,2,4-thiadiazol mit Hydroxyessigsäure-N-methyl-anilid umsetzt.

7. Verfahren nach Anspruch 1, dadurch gekennzeichnet, daß man 3-Trifluormethyl-5-chlor-1,2,4-thiadiazol mit Hydroxyessigsäure-N-methyl-N-cyclohex-1-enyl-amid umsetzt.

**Claims**

1. Process for the preparation of substituted thiadiazolyloxyacetamides of the general formula (I),

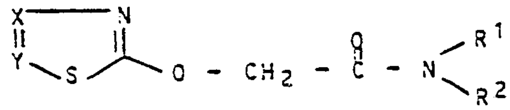

(I)

in which

$R^1$ and $R^2$ independently of one another represent hydrogen, alkyl, alkenyl, alkinyl, optionally substituted cycloalkyl or cycloalkenyl, halogenoalkyl, alkoxyalkyl and alkoxy, aralkyl and optionally substituted aryl, or

$R^1$ and $R^2$, together with the nitrogen atom to which they are bonded, represent an optionally substituted, saturated or unsaturated heterocyclic structure, which can contain further hetero atoms and one of X and Y represents a nitrogen atom and the other the C-$R^3$ grouping wherein

$R^3$ represents alkyl, alkoxy, alkylthio, alkylsulphonyl, halogenoalkyl, aralkyl, aralkoxy, aralkylthio or optionally substituted aryl,

by reacting substituted 5-halogenothiadiazoles of the formula (II)

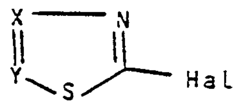

(II)

in which

X and Y have the abovementioned meaning and

Hal represents halogen,

with hydroxyacetamides of the formula (III)

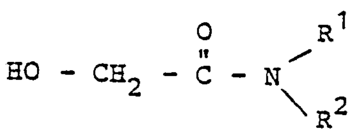

(III)

in which $R^1$ and $R^2$ have the abovementioned meaning, in the presence of a base as an acid acceptor and if appropriate in the presence of a diluent, characterised in that lithium hydroxide or its hydrate is used as the base and the reaction is carried out at temperatures between − 10 °C and + 60 °C.

2. Process according to Claim 1, characterised in that the reaction is carried out at temperatures between + 20 °C and + 40 °C.

3. Process according to Claim 1, characterised in that per mol of 5-halogenothiadiazole of the formula (II) 0.9 to 1.2 mol of hydroxyacetamide of the formula (III) and 0.9 to 1.2 mol of lithium hydroxide or its hydrate are used.

4. Process according to Claim 1, characterised in that 2-trifluoromethyl-5-chloro-1,3,4-thiadiazole is reacted with hydroxyacetic acid 2-ethyl-piperidide.

5. Process according to Claim 1, characterised in that 2-trifluoromethyl-5-chloro-1,3,4-thiadiazole is reacted with hydroxyacetic acid 2-methyl-piperidide.

6. Process according to Claim 1, characterised in that 3-dichlorofluoromethyl-5-chloro-1,2,4-thiadiazole is reacted with hydroxyacetic acid N-methyl-anilide.

7. Process according to Claim 1, characterised in that 3-trifluoromethyl-5-chloro-1,2,4-thiadiazole is reacted with hydroxyacetic acid N-methyl-N-cyclohex-1-enylamide.

**Revendications**

1. Procédé de production de thiadiazolyloxyacétamides substitués de formule générale (I),

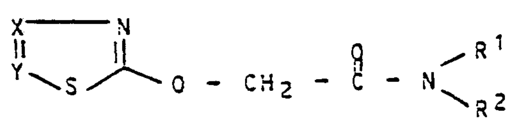

(I)

dans laquelle

$R^1$ et $R^2$ représentent, indépendamment l'un de l'autre, l'hydrogène, un groupe alkyle, alcényle,

alcynyle, un groupe cycloalkyle ou cycloalcényle éventuellement substitué, un groupe halogénalkyle, alkoxyalkyle et alkoxy, un groupe aralkyle et un groupe aryle éventuellement substitué, ou bien

$R^1$ et $R^2$ forment conjointement avec l'atome d'azote auquel ils sont liés un hétérocycle saturé ou insaturé éventuellement substitué, qui peut comporter d'autres hétéro-atomes, et

X et Y peuvent représenter chacun ou bien un atome d'azote ou bien le groupement $C\text{-}R_3\text{-}$, où

$R^3$ est un radical alkyle, alkoxy, alkylthio, alkylsulfonyle, halogénalkyle, aralkyle, aralkoxy, aralkylthio ou aryle éventuellement substitué,

par réaction de 5-halogénothiadiazoles substitués de formule (II)

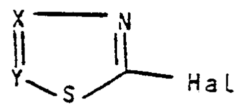

(II)

dans laquelle

X et Y ont la définition indiquée ci-dessus et

Hal représente un halogène,

avec des hydroxyacétamides de formule (III)

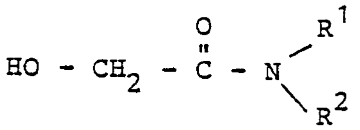

(III)

dans laquelle $R^1$ et $R^2$ ont la définition indiquée ci-dessus, en présence d'une base comme accepteur d'acide et, le cas échéant, en présence d'un diluant, caractérisé en ce qu'on utilise comme base l'hydroxyde de lithium ou son hydrate et on opère à des températures comprises entre − 10 et + 60 °C.

2. Procédé suivant la revendication 1, caractérisé en ce qu'on conduit la réaction à des températures comprises entre + 20 °C et + 40 °C.

3. Procédé suivant la revendication 1, caractérisé en ce qu'on utilise par mole de 5-halogénothiadiazole de formule (II) 0,9 à 1,2 mole d'hydroxyacétamide de formule (III) et 0,9 à 1,2 mole d'hydroxyde de lithium ou de son hydrate.

4. Procédé suivant la revendication 1, caractérisé en ce qu'on fait réagir le 2-trifluorométhyl-5-chloro-1,3,4-thiadiazole avec le 2-éthylpipéridide de l'acide hydroxyacétique.

5. Procédé suivant la revendication 1, caractérisé en ce qu'on fait réagir le 2-trifluorométhyl-5-chloro-1,3,4-thiadiazole avec le 2-méthylpipéridide d'acide hydroxyacétique.

6. Procédé suivant la revendication 1, caractérisé en ce qu'on fait réagir le 3-dichlorofluorométhyl-5-chloro-1,2,4-thiadiazole avec le N-méthylanilide d'acide hydroxyacétique.

7. Procédé suivant la revendication 1, caractérisé en ce qu'on fait réagir le 3-trifluorométhyl-5-chloro-1,2,4-thiadiazole avec le N-méthyl-N-cyclohex-1-énylamide d'acide hydroxyacétique.